# EUROPEAN PATENT SPECIFICATION

(11) **EP 0 753 299 B1**
(45) Date of publication and mention of the grant of the patent: **09.01.2002**
(21) Application number: 95304911.1
(22) Date of filing: 13.07.1995
(51) Int. Cl.: A61K 31/49

(54) **A formulation for iron chelation and a process for preparing same**
Eisen-Chelatierformulierung und Verfahren zu ihrer Herstellung
Formulation de chélation de fer et son procédé de préparation

(43) Date of publication of application: 15.01.1997
(73) Proprietor: Council of Scientific and Industrial Research, New Delhi 110 001 (IN)
(72) Inventor: Sarkar Ajit Kumar, New Dehli-110012 (IN); Kumar Sudarshan, New Dehli-110012 (IN); Priyadarshi Harsh, Allahabad, U.P. (IN); Khanna Sushil Rattan, Rohini Dehli (IN); Dass Ghansham, Ashok Vihar Dehli 110 052 (IN)
(74) Representative: Allden, Thomas Stanley

(56) References cited:
- GARNIER G.; BEZANGER-BEAUQUESNE L.; DEBRAUX G. 'RESSOURCES MEDICINALES DE LA FLORE FRANCAISE' 1961 , PARIS, VIGOT FRERES; FR * page 425 - page 427 *
- HEINZ A. HOPPE 'DROGENKUNDE' 1958 , GRAM, DE GRUYTER & CO , HAMBURG * page 73-74 *

## Description

This invention relates to a pharmaceutical formulation for treating thalassemia.

Thalassemia is a genetic disease among children. It is caused by hereditary disorders connected with defective haemoglobin synthesis, characterized by hypochromia, microcytosis, haemolysis and a variable degree of anaemia. Thalassemia involves a heterogeneous group of molecular defects and presents with a wide spectrum of clinical expressions.

Patients suffering from thalassemia have anaemia with a decreased amount of, or absence of, synthesis of the globin chain of normal haemoglobin. Thalassemia patients are broadly classified into two major groups according to the affected globin chain. Patients suffering from alpha (α) thalassemia have a decreased amount of, or absence of, α- chain synthesis. Patients suffering from beta (β) thalassemia have a decreased amount of, or absence of, β- chain synthesis.

Rarely patients also suffer from thalassemia delta (δ) and gamma (γ) chain disorders, or from disorders associated with abnormal haemoglobin structure (e.g. Hb Lepose and Hb Constant Spring). These disorders also contribute to the thalassemia syndrome.

The disease thalassemia occurs world wide with a particularly high incidence in the Mediterranean basin and in South-East Asia. Malaria is also endemic in these areas - a significant fact since indirect evidence suggests that heterozygosity for thalassemia confers protection against malaria.

The β-thalassemia of thalassemia major type of the disease comprises a heterogeneous group of disorders usually characterised by absence of (β°) or decreased (β⁺) globin synthesis.

The type of β-thalassemia is also classified according to the severity of the anaemia. This clinical classification serves to differentiate homozygous (thalassemia intermedia or thalassemia major) from heterozygous (thalassemia minima or thalassemia minor) conditions. Patients suffering from thalassemia minor have a reduced rate of β- globin synthesis, with an increase in α - β globins chains, but this condition is not a threat to a normal life.

Thalassemia major also known as Cooley's anaemia, Mediterranean anaemia and Von-Jacksch's anaemia is characterised by marked anaemia (ranging from 1 to 6 gm/dl of haemoglobin), severe haemolysis and ineffective erythropoiresis. The diagnosis is made before the patient is one year old, often as early as 3 months old. In patients suffering from thalassemia major, iron in the haemoglobin also breaks down and gets deposited in the vital organs of the patient's body e.g. liver, kidney, spleen, heart etc. This is also known as iron overloading. The life span of a child suffering from thalassemia major is unpredicatable. Every year out of 100,000 children born with thalassemia major in the world, 10,000 are born in India.

The method which has been hitherto available for the treatment of thalassemia major consists of regular blood transfusions coupled with daily intermusculary injections of the drug "Desferal". The chemical name of this drug is deferoxamine methane sulphonate and the chemical formula is C₂₈H₅₂N₄O₁₁S. Desferal causes removal of iron from the patient's body through urinary excretion. It has been found that Desferal has many side effects such as swelling of limbs, stiffness in joints, and may inhibit a number of types of tumour cell proliferation, parasite growth and the proliferation of the cerebral malarial parasite, Plasmodium faliparum.

In addition, the above drug is injected daily under the skin of the patient in a controlled manner in such a way as to avoid any allergic reactions. Such a treatment is very painful. The treatment is also costly as 500 mg of dry active substance costs about US $ 10/-.

Though a new oral iron chelating drug known as L-1, CP20, DMPH, Deferiprone (chemical name 1,2-dimethyl-3-hydroxypyridine-4-one) has been reported very recently, its potential for treating patients suffering from the disease of thalassemia is not yet established. The reported side effects of this drug are 1.myelotoxicity i.e. occurrence of neutropenia, 2. orthropathy i.e. skeleto-muscular pain and swelling around knee and hip joints and lastly mild zinc deficiency occasionally leading to dermatopathy (reference may be made to the Proceedings of National Thalassemia Conference, 5-6 February, 1994, held in Delhi; L1: Oral Iron Chelation Therapy - Indian study by M.B. Agarwal). The cost of a capsule of Defriprone is approximately 50 U.S. cents (Rs. 12/-). A patient has to take 3-6 capsules per day. This amounts to US $ 70-90 (Rs. 1000-2000) per month.

Another method which is available for the treatment of thalassemia major is bone marrow transplantation (BMT). This is done by taking bone marrow of the matching donor and injecting it into the patient. The cost of such a treatment is around US $ 50000/- (Rs. 15,00,000/-). This is therefore beyond the reach of most patients. Several treatments using bone marrow have been performed in many parts of the world including the U.S.A. and Italy. Recently, Christian Medical College (Vellore), and Appolo Hospital (Madras) have also started this type of BMT treatment in India, but the cost is also on the high side (Rs 7 Lac. approx.). Further, increase in life expectancy of these patients has yet to be established.

At present, regular blood transfusion and use of the drug "Desferal" by injection as explained above, is the best way of treatment of this disease. For a decade, efforts have been made by the medical profession throughout the world to find a drug to treat this disease which is of low cost, can be administered orally and has no side effects, but there has been no success so far.

Therefore, the main objective of the present invention is to provide a formulation useful for iron-chelation for the treatment of thalassemia.

Another objective of the present invention is to provide a formulation useful for the treatment of thalassemia with increased therapeutic efficacy.

Yet another objective of the present invention is to provide a formulation for the treatment of thalassemia which is much cheaper and hence affordable by most patients.

Still another objective of the present invention is to provide a formulation useful for the treatment of patients suffering from thalassemia which can be administered orally.

A further objective of the present invention is to provide a formulation which has no side effects and is easy to administer.

Another objective of the present invention is to provide a formulation useful for the treatment of thalassemia, the administration of which can be continued for a long period without damaging any of the patient's vital organs.

It has been observed that patients suffering from thalassemic disease have a change in complexion, anorexia, blackness of gums, increase in serum ferritin level and a severe iron overload in the body due to the breaking down of red blood cells. The treatment mentioned above which involves repeated blood transfusion, also accumulates iron in the patient's body.

Children suffering from thalassemia disease show no resistance to viral infection and also suffer from body ache. They are also immune to malaria.

Our research work was primarily directed to finding an immediate solution for the survival of such patients, who had an iron overload in the body and the other side ailments mentioned above.

Efforts, therefore, were directed towards finding an alternative drug which can be used orally and has no side effects. Anemonin pretensis (powder) has been used for many years by tribes of Siberia to poison their arrows. Anemonin pretensis is extracted with an organic solvent such as ethanol from fresh whole wind flower plant. The dried material is treated with an organic solvent such as ethanol and refluxed. Anemonin pretensis obtained from the extraction is filtered and recrystallised from an organic solvent such as ethanol. The medical history of this herb also reveals that this herb was involved in homeopathic practice in 1805 for female hormone problems.

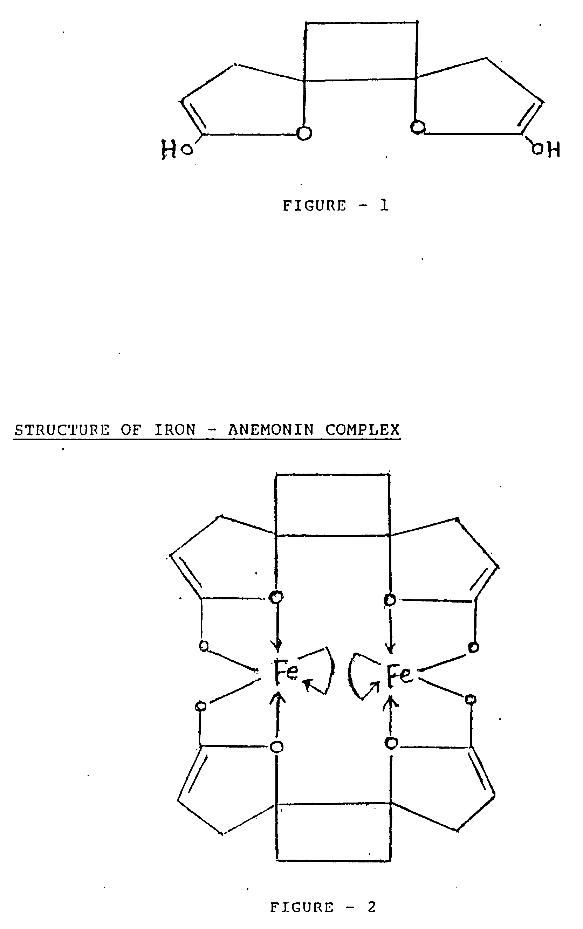

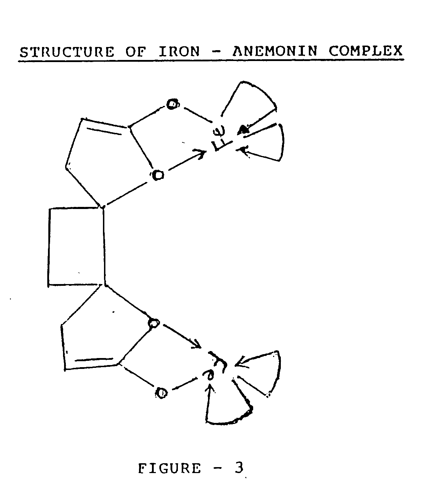

The chemical formula of anemonin pretensis having the formula 1, shown in the drawing accompanying this specification, is 1,2-dihydroxy-1,2-cyclobutane diacrylic acid di-lactone. Reference may be made to Merck index P-87, Entry no. 677, 9th edition.

Patients suffering from thalassemia disease are immune to malarial attack. In view of this, we have considered incorporating quinine sulphate, a known antimalarial drug having antipyretic and analgesic properties, in the formulation of the invention to reduce high temperature and body ache in patients suffering from thalassemic disease.

Quinine has the chemical composition C₂₀H₂₄N₂O₂. It is obtained from Cinchona bark. Quinine is extracted from the bark with an organic solvent such as ethanol. This is then refluxed, filtered and the quinine recrystallized from an organic solvent such as ethanol. Quinine sulphate is prepared by reacting quinine and dilute sulphuric acid in a 2:1 molar ratio as shown below: 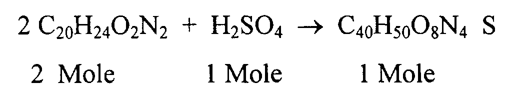 Quinine sulphate exists as snow-white, light, odourless, extremely bitter crystallized needles having the chemical composition C₄₀H₄₈N₄O₄, H₂SO₄, H₂O. Reference may be made to Merck Index p-1049, entry no. 7879, 9th edition.

During our research work we observed that when we blended (preferably mechanically) anemonin pretensis powder with quinine sulphate and dissolved it in a suitable solvent, and the solution was administered orally to a patient suffering from thalassemia, there was a remarkable improvement of complexion and reduction of the serum ferritin level. There was no fever. There was also no body ache. The treatment was continued and more patients were put on a trial of this drug. There were no side effects. This showed that the formulation is very useful in the treatment of the patients suffering from thalassemia.

Accordingly, the present invention provides a pharmaceutical formulation useful for treating patients suffering from thalassemia which comprises:
i. powder of anemonin pretensis in an amount in the range of 0.02 to 0.12 wt% of the formulation.
ii. quinine sulphate in an amount in the range of 0.0005 to 0.003 wt % of the formulation.
iii. distilled or demineralised water in an amount in the range of 0 to 40 wt % of the formulation, and
iv. edible solvent 99.88 to 60 wt % of the formulation.

The solvent used may be selected from ethanol, absolute alcohol etc.

The anemonin pretensis powder and the quinine sulphate employed in the formulation may be of pharmaceutical grade.

Anemonin pretensis possesses iron-chelating properties. Quinine sulphate seems to accelerate the chelation of iron in the patient's body. We have found that forming a formulation of anemonin pretensis and quinine sulphate in the amounts mentioned above results in unexpected properties which are not present in the individual components. There is, therefore, a synergistic activity when they are combined in the above mentioned quantities.

The formulation of the present invention is, therefore, not a mere admixture of the ingredients employed but a synergistic mixture, the properties of which are not merely the aggregate properties of the individual ingredients of the formulation.

The formulation of the present invention when administered to patients suffering thalassemic disease works as follows:

The iron present in the patient's body forms a complex with anemonin, as shown in Formula 2 of the drawings (closed ring structure) or in Formula 3 (stretched structure), wherein three molecules of anemonin can form a complex with two iron atoms. The quinine sulphate present in the formulation acts as a catalyst for the formation of the complex thereby not only reducing the amount of anemonin in the complex/composition but also using the maximum amount of iron present in the body. In addition, the antipyretic and analgesic properties of quinine sulphate help to control the fever and the body ache problem present in such patients.

The above-described iron complex is formed in the body fluids. The fluid containing the complex reaches the kidneys through the blood system and it is then excreted from the body in the urine. Some of the complex is also excreted via the alimentary canal and faeces. By these processes the excess iron present in the patient's body is removed, thereby enhancing the life span of the patient. The various ingredients of the present formulation can be blended by any conventional method such as mechanical mixing etc.

The following examples are given by way of illustration.

### Example 1

A formulation was prepared by blending the following ingredients:

| | |
|---|---|
| powder of Anemonin Pretensis | 0.02 wt% of the formulation |
| quinine Sulphate | 0.0005 wt % of the formulation |
| demineralised Water | 10 wt % of the formulation |
| ethanol Solvent | 89.9795 wt % of the formulation |

### Example 2

A formulation was prepared by blending the following ingredients:

| | |
|---|---|
| powder of Anemonin Pretensis | 0.04 wt% of the formulation |
| quinine Sulphate | 0.0008 wt % of the formulation |
| demineralised Water | 10 wt % of the formulation |
| ethanol Solvent | 89.9592 wt % of the formulation |

### Example 3

A formulation was prepared by blending the following ingredients:

| | |
|---|---|
| powder of Anemonin Pretensis | 0.06 wt% of the formulation |
| quinine Sulphate | 0.001 wt % of the formulation |
| demineralised Water | 10 wt % of the formulation |
| ethanol Solvent | 89.939 wt % of the formulation |

### Example 4

A formulation was prepared by blending the following ingredients:

| | |
|---|---|
| powder of Anemonin Pretensis | 0.08 wt% of the formulation |
| quinine Sulphate | 0.002 wt % of the formulation |
| demineralised Water | 10 wt % of the formulation |
| ethanol Solvent | 89.918 wt % of the formulation |

### Example 5

A formulation was prepared by blending the following ingredients:

| | |
|---|---|
| powder of Anemonin Pretensis | 0.09 wt% of the formulation |
| quinine Sulphate | 0.001 wt % of the formulation |
| demineralised Water | 10 wt % of the formulation |
| ethanol Solvent | 89.919 wt % of the formulation |

### Example 6

A formulation was prepared by blending the following ingredients:

| | |
|---|---|
| powder of Anemonin Pretensis | 0.12 wt% of the formulation |
| quinine Sulphate | 0.003 wt % of the formulation |
| demineralised Water | 10 wt % of the formulation |
| ethanol Solvent | 88.8779 wt % of the formulation |

### Example 7

A formulation was prepared by blending the following ingredients:

| | |
|---|---|
| powder of Anemonin Pretensis | 0.09 wt% of the formulation |
| quinine Sulphate | 0.001 wt % of the formulation |
| demineralised Water | 80 wt % of the formulation |
| ethanol Solvent | 19.909 wt % of the formulation |

### Example 8

A formulation was prepared by blending the following ingredients:

| | |
|---|---|
| powder of Anemonin Pretensis | 0.08 wt% of the formulation |
| quinine Sulphate | 0.001 wt % of the formulation |
| demineralised Water | 40 wt % of the formulation |
| ethanol Solvent | 59.919 wt % of the formulation |

The formulations mentioned in Examples 1 to 8 were administered to patients of different age groups who were suffering from thalassemia. The patients were administered the formulation orally after mixing it with water. The formulation was administered to the patient daily. This treatment was continued for a period of two months. All the formulations caused iron to be excreted in the urine at a range from 10-95 %. This result indicates that the formulation of the present invention is very effective for the chelation of iron, thereby reducing the serum ferritin level in the patients suffering from thalassemia. This results in longer survival and larger survival rate of the patients. The above mentioned results are shown in Table 1.

**Table-1**

| **Efficiency of the formulation of the present invention** | | | | | | | |
|---|---|---|---|---|---|---|---|
| Formulation used | No. of patients treated | Age (yrs) | Sex | Frequency of transfusion (days) | Duration of treatment (months) | Medicine Administered in drops twice daily | Excretion chelated iron (%) |
| Example 1 | 3 | 5 | M | 22 | 2 | 15 | 15 |
| | | 7 | F | | | | 25 |
| | | 7 | F | | | | 25 |
| Example 2 | 3 | 9 | M | 20 | 2 | 15 | 40 |
| | | 9 | M | | | | 25 |
| | | 11 | M | | | | 30 |
| Example 3 | 3 | 7 | M | 21 | 2 | 15 | 40 |
| | | 7 | M | | | | 40 |
| | | 9 | F | | | | 50 |
| Example 4 | 3 | 15 | M | 20 | 2 | 15 | 70 |
| | | 20 | F | | | | 70 |
| | | 23 | F | | | | 80 |
| Example 5 | 3 | 3 | M | 21 | 2 | 15 | 90 |
| | | 9 | F | | | | 91 |
| | | 19 | M | | | | 95 |
| Example 6 | 3 | 10 | M | 22 | 2 | 15 | 20 |
| | | 11 | F | | | | 20 |
| | | 12 | M | | | | 30 |
| Example 7 | 3 | 15 | M | 21 | 2 | 15 | 10 |
| | | 16 | F | | | | 15 |
| | | 19 | F | | | | 10 |
| Example 8 | 3 | 7 | M | 20 | 2 | 15 | 75 |
| | | 9 | M | | | | 75 |
| | | 11 | F | | | | 80 |

**Table - 2**

| **Comparison of Desferal, Defriprone and the formulation of the present invention** | | | |
|---|---|---|---|
| Name of the medicine | Mode of Administration | Side effects | Taste |
| Desferal | Injection (highly painful) | Adverse side effects such as: a. ocular toxicity | Does not apply |
| | | b. auditory meaurotoxicity | |
| | | c. cerebral toxicity | |
| | | d. allergic skin reaction | |
| | | e. cardiovascular and gastrointestinal disturbances | |
| | | f. change in blood pressure | |
| | | | |
| Defriprone | Oral | More side effects than Desferal such as: | Bitter taste |
| | | a. Myelotoxicity | |
| | | b. Arthropathy | |
| | | c. Mild Zinc deficiency | |
| | | d. Questionable occurrence of immunological complications | |
| | | | |
| Formulation of the present invention | Oral | No side effects | Tasteless |

**Table 3**

| **Annual cost of treatment of thalassemia using the hitherto known drugs and the formulation of the present invention** | | |
|---|---|---|
| Name of medicine | Amount in US $ | Reference |
| DESFERAL | 3000 - 6000 | Iron Chelation Therapy C. Hershko & D.J. Weatherall |
| | | |
| | | CRC Critical Reviews Clinical Laboratory Series 26(4), 314, 1988 |
| | | |
| DEFRIPRONE (L-1) | 800 - 1100 | News Review, United Kingdom Thalassemia Soc Issue No. 61, March, 95 |
| | | |
| PRESENT FORMULATION | 50 - 60 | -------- |

Table 3 illustrates that the cost of the present formulation is the cheapest when compared with known drugs.

The formulation of the present invention can be in the form of tablets, powder or suspension.

The main advantages of the formulation of the present invention are:
1. Iron-chelation using the formulation of anemonin pretensis and quinine sulphate is up to 90%.
2. The cost of the formulation is around US$5 to 10/- (Rs. 150-200/-) per 30 ml vial and it can be used for one month. The cost is much lower as compared to other currently available medicines on the market for the treatment of thalassemia.
3. The formulation has no toxic effects even when it is administered for long periods.
4. The formulation is tasteless and odourless and can be administered orally.

## Claims

1. A pharmaceutical formulation useful for treating patients suffering from thalassemia, which comprises:
a. powder of anemonin pretensis in an amount of 0.02 to 0.12 wt % of the formulation,
b. quinine sulphate in an amount of 0.0005 to 0.003 wt % of the formulation,
c. distilled or demineralised water in an amount of 0 to 40 wt % of the formulation and
d. edible solvent 99.88 to 60 wt % of the formulation.

2. A pharmaceutical formulation as claimed in claim 1, wherein the edible solvent is present in an amount of 99.88 to 59.889 wt %.

3. A pharmaceutical formulation as claimed in claim 1 or 2, wherein the anemonin pretensis is present in an amount selected from 0.04 wt %, 0.06 wt %, 0.08 wt % and 0.09 wt %.

4. A pharmaceutical formulation as claimed in claim 1, 2 or 3, wherein the anemonin pretensis used is of pharmaceutical grade.

5. A pharmaceutical formulation as claimed in any of claims 1 to 4, wherein the solvent used is ethanol or absolute alcohol.

6. A pharmaceutical formulation as claimed in any of claims 1 to 5, wherein the quinine sulphate used is of pharmaceutical grade.

7. A pharmaceutical formulation as claimed in any of claims 1 to 6, wherein the quinine sulphate is present in an amount selected from 0.0008 wt %, 0.001 wt %, and 0.002 wt %.

8. A pharmaceutical formulation as claimed in any of claims 1 to 7, wherein the solvent is present in an amount which is selected from 89.9795 wt %, 89.9592 wt %, 89.939 wt %, 89.918 wt %, 89.97 wt %, 89.95 wt %, 88.87 wt %, 89.92 wt %, 89.94 wt%, 19.909 wt % and 59.919 wt %.

9. A process for the preparation of a pharmaceutical composition for treating patients suffering from thalassemia, said process comprising mixing:
a. powder of anemonin pretensis in an amount of 0.02 to 0.12 wt % of the formulation,
b. quinine sulphate in an amount of 0.0005 to 0.003 wt % of the formulation,
c. distilled or demineralised water in an amount of 0 to 40 wt % of the formulation, and
d. edible solvent 99.8 to 60 wt % of the formulation.

10. A process as claimed in claim 9, wherein the anemonin pretensis powder and the quinine sulphate used are of pharmaceutical grade.

11. A process as claimed in claim 9 or 10, wherein the solvent used is ethanol or absolute alcohol.

## Patentansprüche

1. Pharmazeutische Rezeptur, die bei der Behandlung von an Thalassämie leidenden Patienten nützlich ist, welche Rezeptur Folgendes umfasst:
a. pulverförmiges Anemonin pretensis in einer Menge von 0,02 bis 0, 12 Gew.-%, auf die Rezeptur bezogen;
b. Chinisulfat, in einer Menge von 0,0005 bis 0,003 Gew.-% , auf die Rezeptur bezogen;
c. destilliertes oder entmineralisiertes Wasser in einer Menge von 0 bis 40 Gew.-%, auf die Rezeptur bezogen, und
d. 99,88 bis 60 Gew.-% genießbares Lösungsmittel, auf die Rezeptur bezogen.

2. Pharmazeutische Rezeptur nach Anspruch 1, in der das genießbare Lösungsmittel in einer Menge von 99,88 bis 59,889 Gew.-% vorliegt

3. Pharmazeutische Rezeptur nach Anspruch 1 oder 2, in der das Anemonin pretensis in einer Menge vorliegt, die unter 0,04 Gew.-%, 0,06 Gew.-%, 0.08 Gew.-% und 0,09 Gew.-% ausgewählt wird.

4. Pharmazeutische Rezeptur nach Anspruch 1, 2 oder 3, in der das verwendete Anemonin pretensis von pharmazeutischer Qualität ist.

5. Pharmazeutische Rezeptur nach einem der Ansprüche 1 bis 4, in der das verwendete Lösungsmittel Ethanol oder Absolutalkohol ist.

6. Pharmazeutische Rezeptur nach einem der Ansprüche 1 bis 5, in der das verwendete Chininsulfat von pharmazeutischer Qualität ist. ??

7. Pharmazeutische Rezeptur nach einem der Ansprüche 1 bis 6, in der das Chininsulfat in einer Menge vorliegt, die unter 0,0008 Gew.-%, 0,001 Gew.-% und 0,002 Gew.-% ausgewählt wird.

8. Pharmazeutische Rezeptur nach einem der Ansprüche 1 bis 7, in der das Lösungsmittel in einer Menge vorliegt, die unter 89,9795 Gew.-%, 89,9592 Gew.-%, 89,939 Gew.-%, 89,918 Gew.-%, 89,97 Gew.-%, 89,95 Gew.-%, 88,87 Gew.-%, 89,92 Gew.-%, 89,94 Gew.-%, 19,909 Gew.-% und 59,919 Gew.-% ausgewählt wird.

9. Verfahren für die Zubereitung einer pharmazeutischen Zusammensetzung für die Behandlung von an Thalassämie leidenden Patienten, wobei das Verfahren das Mischen umfasst von:
a. pulverförmigem Anemonin pretensis in einer Menge von 0,02 bis 0, 12 Gew.-%, auf die Rezeptur bezogen;
b. Chinisulfat, in einer Menge von 0,0005 bis 0,003 Gew.-% , auf die Rezeptur bezogen;
c. destilliertem oder entmineralisiertem Wasser in einer Menge von 0 bis 40 Gew.-%, auf die Rezeptur bezogen, und
d. 99,88 bis 60 Gew.-% genießbarems Lösungsmittel, auf die Rezeptur bezogen.

10. Verfahren nach Anspruch 9, bei dem das pulverförmige Anemonin pretensis und das Chininsulfat von pharmazeutischer Qualität sind.

11. Verfahren nach Anspruch 9 oder 10, bei dem das verwendete Lösungsmittel Ethanol oder Absolutalkohol ist.

## Revendications

1. Une composition pharmaceutique utile pour traiter des patients atteints de thalassémie, comprenant :
a. poudre d'anémonine pretensis en une quantité de 0,02 à 0,12% en poids de la composition,
b. Sulfate de quinine en une quantité de 0,0005 à 0,003% en poids de la composition,
c. Eau distillée ou déminéralisée en une quantité de 0 à 40% en poids de la composition et
d. Solvant comestible 99,88 à 60% en poids de la composition.

2. Une composition pharmaceutique selon la revendication 1, dans laquelle le solvant comestible est présent en une quantité de 99,88% à 59,889% en poids.

3. Une composition pharmaceutique selon la revendication 1 ou 2, dans laquelle l'anémonine pretensis est présente en une quantité sélectionnée parmi 0,04% en poids, 0,06% en poids, 0,08% en poids et 0,09% en poids.

4. Une composition pharmaceutique selon la revendication 1, 2 ou 3, dans laquelle l'anémonine pretensis utilisée est de qualité pharmaceutique.

5. Une composition pharmaceutique selon l'une quelconque des revendications 1 à 4, dans laquelle le solvant utilisé est l'éthanol ou alcool absolu.

6. Une composition pharmaceutique selon l'une quelconque des revendications 1 à 5, dans laquelle le sulfate de quinine utilisé est de qualité pharmaceutique.

7. Une composition pharmaceutique selon l'une quelconque des revendications 1 à 6, dans laquelle le sulfate de quinine est présent en une quantité sélectionnée parmi 0,0008% en poids, 0,001% en poids et 0,002% en poids.

8. Une composition pharmaceutique selon l'une quelconque des revendications 1 à 7, dans lqauelle le solvant est présent en une quantité sélectionnée parmi 89,9795% en poids, 89,9592% en poids, 89,939% en poids, 89,918% en poids, 89,97% en poids, 89,95% en poids, 88,87% en poids, 89,92% en poids, 89,94% en poids, 19,909% en poids et 59,919% en poids.

9. Un procédé pour la préparation d'une composition pharmaceutique pour traiter des patients atteints de thalassémie, ledit procédé comprenant le mélange de :
a. poudre d'anémonine pretensis en une quantité de 0,02 à 0,12% en poids de la composition,
b. sulfate de quinine en une quantité de 0,0005 à 0,003% en poids de la composition,
c. eau distillée ou déminéralisée en une quantité de 0 à 40% en poids de la composition, et
d. solvant comestible 99,8 à 60% en poids de la composition.

10. Un procédé selon la revendication 9, selon lequel la poudre d'anémonine pretensis et le sulfate de quinine utilisés sont de qualité pharmaceutique.

11. Un procédé selon la revendication 9 ou 10, selon lequel le solvant utilisé est l'éthanol ou alcool absolu.
